(19) Europäisches Patentamt

European Patent Office

Office européen des brevets

(11) **EP 0 655 861 B1**

## (12) EUROPEAN PATENT SPECIFICATION

(45) Date of publication and mention
of the grant of the patent:
**02.08.2000 Bulletin 2000/31**

(51) Int. Cl.⁷: **H04N 5/32**

(21) Application number: **94203387.9**

(22) Date of filing: **22.11.1994**

(54) **Image composition method and imaging apparatus for performing said method**

Verfahren zur Bildzusammensetzung und Bilderzeugungsvorrichtung zur Durchführung dieses
Verfahrens

Méthode de composition d'image et appareil de formation d'image pour la mise en oeuvre de cette
méthode

(84) Designated Contracting States:
**DE FR GB NL**

(30) Priority: **26.11.1993 EP 93203320**

(43) Date of publication of application:
**31.05.1995 Bulletin 1995/22**

(73) Proprietor:
**Koninklijke Philips Electronics N.V.
5621 BA Eindhoven (NL)**

(72) Inventors:
• **Lobregt, Steven
NL-5656 AA Eindhoven (NL)**
• **van Eeuwijk, Alexander Henricus Waltherus
NL-5656 AA Eindhoven (NL)**

(74) Representative:
**Schouten, Marcus Maria
INTERNATIONAAL OCTROOIBUREAU B.V.,
Prof. Holstlaan 6
5656 AA Eindhoven (NL)**

(56) References cited:
**EP-A- 0 166 635          EP-A- 0 576 066
WO-A-90/02466          US-A- 4 562 464
US-A- 4 644 575          US-A- 5 123 056**

• **PATENT ABSTRACTS OF JAPAN vol. 17, no. 496
(C-1108) 8 September 1993 & JP-A-05 130 984
(TOSHIBA K.K.) 28 May 1993**

**Description**

The invention relates to an image composition method comprising the steps of making a series of consecutive sub-images each representing a portion of an elongate scene, which sub-images overlap along the elongate scene, and of merging the sub-images into an assembled image. The invention also relates to x-ray examination apparatus comprising an image processor for performing said method.

An image composition method of said kind and a device for performing this method are described in the European Patent Application **EP 0 576 066** which is comprised in the state of the art pursuant to Art. 54(3)EPC. An image composition method and a device for performing such a method are also known from the United States Patent **US 5 123 056**. Further the United States Patent **US 4 644 575** discloses that an image is composed from successive x-ray shadow images of a slit. Further, the Japanese patent application no. **JP 05-130 984** discloses an X-ray CT device which overlap scans the boundaries of images and an interpolation processing is executed to the data obtained by this overlap scan.

The United States Patent **US 5 123 056** in particular discloses an image composition method for use in peripheral angiography by means of x-ray radiography. The x-ray examination apparatus described in the cited reference comprises an x-ray source, an x-ray detector facing the x-ray source and a patient table. The patient table on the one hand and the x-ray source and the x-ray detector on the other hand are displaceable with respect to one another. Consecutive sub-images of adjacent portions of a patient's limb are made by successive irradiations while the patient is translated *vis-à-vis* the x-ray source over some distance between successive irradiations. X-ray imaging is performed on the patient's limb in peripheral angiography. Because of the size of the limb of interest, an x-ray examination apparatus is unable to image the entire limb at once during a single irradiation step. Instead, a number of x-ray sub-images are made of a patient's limb that is to be examined. These x-ray sub-images are converted into electronic sub--images by means of an x-ray image detector in the form of an image intensifier television chain that is included in the x-ray examination apparatus. In order to form a single image of entire limb the electronic sub-images are processed in such a way that overlapping portions of said electronic sub-images are deleted. Subsequently from said processed electronic sub-images an assembled image is formed by adjoining together these consecutive processed electronic sub-images. The assembled image is supplied in the form of an electronic video signal which may be supplied to a monitor for direct viewing or to a device for forming a hard-copy of the assembled image.

In the image composition method of the cited reference the overlapping portions of consecutive sub-images are calculated from positions of a carrier to which the x-ray source and the x-ray detector are mounted, and from positions of the patient table of the x-ray apparatus during x-irradiation for the formation of each of the x-ray sub-images. Thus, the known image assembly method can be employed only if data on the positions of the x-ray source, x-ray detector and patient table are recorded together with the x-ray sub-images. It is common practice to fit an x-ray examination apparatus with a device for measuring and recording such positions. Consequently the known method suffers from a substantial drawback in that the known method is unsuitable for forming an assembled image from sub-images produced by an x-ray examination apparatus which does not record positions of the carrier and the patient table. In each of the sub-images moreover image-distortions arise, such as pincushion-distortion caused by the curvature of the entrance screen of the image intensifier, S-distortion caused by stray magnetic fields which perturb the electron-optics of the image intensifier, vignetting caused by to the spatial variations across the x-ray beam and due to differences in the size of the patient's body. Furthermore, structures inside the patients' limbs, in particular bones, arteries and veins, are imaged with a parallax which varies among the sub-images. Consequently, the assembled image formed by the known assembly method contains disturbing transitions at the boundaries of the processed sub-images as well as substantial pin-cushion distortions which vary over the entire assembled image. These image distortions have a deleterious effect on the diagnostic quality of the assembled image made by the conventional assembly method.

It is, *inter alia*, an object of the invention to provide an image composition method such that the influence on the assembled image with artifacts caused by assembly of sub-images is substantially counteracted. It is also an object of the invention to provide an x-ray examination apparatus which is suitable for performing the image composition method according to the invention.

This object is achieved by the image processing method according to the invention as defined in Claim 1.

An elongate scene may e.g. consist of an x-ray shadow image of a patient's limb or of a magnetic resonance image of a patient's spine. The elongate scene is imaged by a series of consecutive sub-images. According to the image composition method according to the invention mutually corresponding pixel-values each pertaining to the same position in the elongate scene are selected from overlapping portions of consecutive sub-images and are interpolated to form a pixel-value for the position in the elongate scene. It is achieved in this way that sudden transitions in the assembled image at boundaries of regions pertaining to adjacent sub-images are substantially avoided. A pixel-value of a pixel of the assembled image which corresponds to a portion of the elongate scene imaged in overlapping portions of sub-images, is formed from pixel-values of corresponding pixels of the relevant consecutive sub-images in that a value between the respective pixel-values from the sub-images is supplied through interpolation by a predetermined interpolation function. This interpolation function may e.g. have the form of a weighted sum of the pixel-values from the sub-images.

Especially when the series of sub-images consists of sub-images made by x-irradiation for examination of a patient, the avoidance of such sudden transitions substantially improves the assembled image for use in medical examinations because the assembled image does not contain artifacts formed by said transitions which may obscure lesions, or may inadvertently show an image similar to a lesion whereas the imaged portion of the patient does not comprise any lesions whatsoever. Furthermore, the sub-images may alternatively be formed from a magnetic resonance imaging examination or from an ultrasound imaging examination. Such imaging modalities may image an elongate scene by forming a series of consecutive sub-images which overlap the elongate scene. It is advantageous for x-ray examinations as well as for magnetic resonance imaging and ultrasound imaging for diagnostic purposes, to form an assembled image from a series consecutive sub-images which contain much redundant image information.

In order to match accurately consecutive sub-images when they are merged into the assembled image, the shift with respect to the elongate scene between consecutive sub-images is determined. When said shift has been determined, the consecutive sub-images can be merged into the assembled image in such a way that the assembled image represents the scene in a continuous way such that there are no portions deleted between consecutive images and there are no duplicate portions in the assembled image. In the implementation of the method in accordance with the invention, said relative shift is determined from the image information contained in the sub-images themselves so that it is not required to perform any measurement relating to positioning of e.g. an image acquisition means relative to the elongate scene, when the sub-images are being formed. In particular said shift is computed from a comparison pixel-values of pixels in overlapping portions of respective consecutive sub-images which correspond to the same position in the elongate scene.

In order to accurately calculate said shift between consecutive sub-images there are determined correlations of pixel-values of pixels in overlapping portions of respective consecutive sub-images and corresponding to the same position in the elongate scene. Such correlations preferably take the form of an average-value of the product of pixel-values of pixels from consecutive sub-images. The correlations are in particular calculated as a function of shift-values of the relative shift between consecutive sub-images. The actual value of said relative shift is subsequently obtained as the shift-value corresponding to the maximum value of the correlations between consecutive sub-images.

When sub-images which are to be merged contain little contrast, the determination of correlations between pixel-values on overlapping portions of consecutive sub-images is improved by the intentional addition of contrast in the sub-images. The contrast in the sub-images is enhanced in accordance with the invention by imaging a contrasting object together with the elongate scene when the elongate scene itself comprises comparatively little contrast. Especially when making sub-images in peripheral angiography the elongate scene may comprise too little contrast to render possible accurate determination of said correlations from the representation of the elongate scene in the sub-images only. Imaging a contrasting object together with the actual elongate scene adds a sufficient amount of contrast to each of the sub-images to enable accurate calculations of said relative shift from correlations.

A preferred implementation of an image composition method according to the invention is characterized in that the contrasting object has the form of a ruler comprising a binary pattern.

A binary pattern comprises two distinct features inducing contrast. The use of a binary pattern achieves that the contrasting object on the one hand induces a sufficient amount of contrast in the sub-images for enabling accurate determination of said correlations, while on the other hand any obscuration of the elongate scene which is to be represented in the assembled image is substantially limited. It has been found that the contrasting object preferably has the form of a ruler comprising a binary pattern; i.e. the contrasting object comprises a grid pattern of two predetermined distinct features. The use of two features adds contrast to the elongate scene, while it is avoided that spurious correlations are induced. The presence of only two features achieves that the elongate scene as such is obscured to a minimal extent and since the two features are predetermined there is minimal interference with features in the elongate scene when one examines an assembled image representing both the elongate scene as such and the contrasting object is inspected.

An x-ray examination apparatus which is suitable for performing the image composition method according to the invention preferably incorporates a patient support which incorporates a ruler comprising a binary pattern of x-ray absorbing portions and x-ray transmitting portions.

A further preferred implementation of an image composition method according to the invention is characterized in that the ruler has a planar shape and comprises an elongate central feature and first protrusions extending from the central feature in a first direction, and second protrusions extending from the central feature in a second direction while the pitch between adjacent first protrusions is substantially equal to the pitch between adjacent second protrusions and the spacing between adjacent first protrusions are offset with respect to the spacing between adjacent second protrusions.

The shape of the ruler is such that the distance between consecutive first protrusions and between consecutive second protrusions is comparatively large, but because of an offset between the succession of first protrusions and the succession of second protrusions the distance between two protrusions of different type is smaller than the distance between consecutive protrusions of the same type. The ruler may be comparatively small subject to the choice of a size of the sub-images or of portions of the sub-images used for calculation of the shift between consecutive sub-images; nevertheless there will be always one protrusion of either the first type or the second type which is be imaged in each of said possible partial sub-images, but it may be excluded that there are two protrusions of the same kind imaged in each of said possibly partial sub-images. Consequently, the ruler substantially suppresses the introduction of spurious correlations by the ruler. The protrusions may have the shape of bars extending from the central feature; or alternatively patterns may be employed having a shape being distinct from features in the elongate scene.

An x-ray examination apparatus comprising an image processor for performing an implementation of the image composition method according to the invention preferably comprises a patient support which incorporates a ruler which has a planar shape and comprises an elongate central feature and first protrusions extending from the central feature in a first direction, and second protrusions extending from the central feature in a second direction and the pitch between adjacent first protrusions being substantially equal to the pitch between adjacent second protrusions and the spacing between adjacent first protrusions being offset with respect to the spacing between adjacent second protrusions.

When consecutive sub-images are made by any imaging means, image distortions often are most abundant away from the centre of each of the sub-images. Such image distortions occur especially when sub-images are made by x-irradiation and x-ray sub-images into visible sub-images are converted by means of an x-ray image intensifier. Notably, each consecutive sub-image shows pin-cushion distortions away from the centre of the sub-image. When an elongate scene is imaged by x-irradiation with the x-ray source and detector being moved along the elongate scene, e.g. along a patient's limb, features in the elongate scene, in this case in the patient's limb, are imaged with a parallax which varies along the direction of motion of the x-ray source. According to the invention, portions of the sub-images are each selected near the centre of a relevant sub-image and said selected portions are employed for the merging into an assembled image whereby pin-cushion distortions are substantially avoided in the assembled image. Moreover, image distortions caused by parallax differences between sub-images are substantially reduced because only portions of sub-images which have parallax-differences in only one direction, viz. perpendicular to the longitudinal axis of the elongate scene are employed in merging. Any remaining perturbations caused by parallax are minimised in the assembled image because in the selected portions of the sub-images the parallax is smaller than in portions of the sub-images outside the selected portions. In order to obtain a sufficient amount of overlap of respective selected lengthened portions the width of the lengthened portions is preferably chosen to be substantially twice the value of the shift between the consecutive sub-images from which the lengthened portions have been selected.

An x-ray examination apparatus comprising an x-ray source for emitting an x-ray beam, an x-ray detector facing the x-ray source, the apparatus comprising a patient-support, the x-ray source, the x-ray detector and the patient-support being moveable with respect to one another so as to enable the formation of a series of consecutive images of an elongate portion of the patient and by x-irradiation of successive portions of the patient, preferably comprises an image processor which is suitable for performing the image composition method according to the invention.

A preferred embodiment of an x-ray examination apparatus according to the invention is defined in Claim 5.

X-ray absorption means, such as positionable x-ray shutters substantially block out portions of the x-ray beam so that a controlled portion of a patient is x-irradiated. It is advantageous to employ the x-ray absorption means for selecting the lengthened portions of the sub-images that are actually used in the formation of the assembled image because it enables a reduction in the x-ray dose to which the patient is exposed during a radiological examination.

These and other aspects of the invention will become apparent from and will be elucidated with reference to the implementations and embodiments described hereinafter and with reference to the accompanying drawings, in which

Figure 1 shows a side elevation of an x-ray examination apparatus in accordance with the invention,
Figure 2 shows a diagrammatic representation of the formation of sub-images and the assembly of an assembled image from the consecutive sub-images,
Figure 3 shows an example of a sub-image which is involved in the assembly of an assembled image,
Figure 4 illustrates the choice of a gauge vector from a first sub-image and of a template vector from a second sub-image,
Figure 5 illustrates the merging of sub-images into an assembled image in accordance with the invention,
Figures 6a and 6b illustrate respective embodiments of contrasting objects having the form of a planar ruler having a binary pattern.

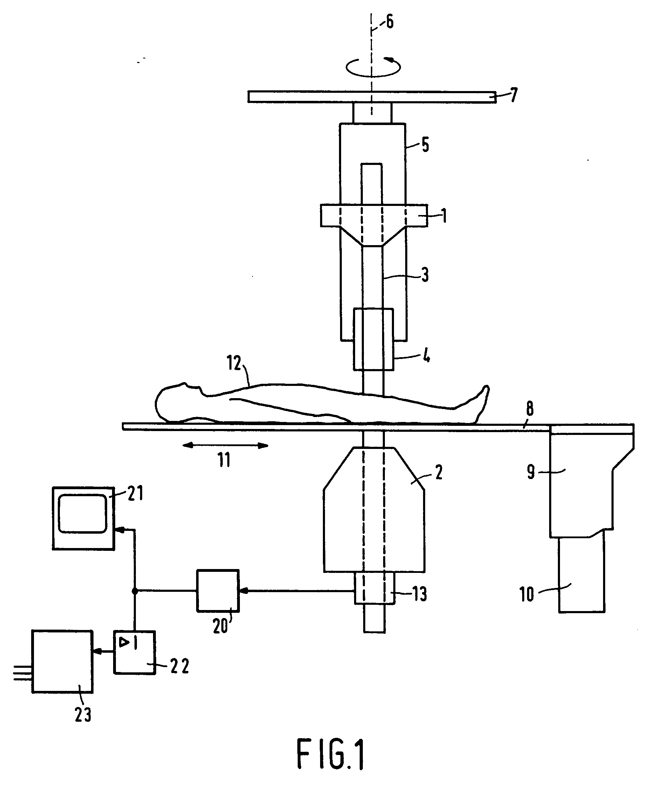

Figure 1 shows a side elevation of an x-ray examination apparatus in accordance with the invention. An x-ray source 1 and an x-ray detector 2, e.g. an x-ray image intensifier, are connected to a carrier 3, e.g. a C-arm. The C-arm is movably mounted to a predominantly vertical support 5 by means of a sleeve 4. The vertical support is rotatable around a predominantly vertical axis 6 of rotation and the vertical support is mounted to a system of guide rails 7 which are attached to the ceiling of the room in which the x-ray apparatus is installed. The patient table 8 is mounted to a table frame 9 which is mounted to a pillar 10. The frame is moveable along the pillar, so as to adjust the height of the patient table above the x-ray source. The table 8 is also movably connected to the frame so as to allow longitudinal 11 displacement of the patient table with the patient 12. The x-ray image intensifier 2 converts an x-ray image formed on the entrance screen of the image intensifier into a light-image on the output window of the image intensifier. The light-image is registered by a system of lenses and a videocamera 13. The videocamera 13 provides an electronic video signal for each of the registered images, which videosignais are supplied to an image processor 20 which merges electronic sub-images into an assembled image in the form of an electronic image signal which may be supplied to a monitor 21 for viewing or to a buffer circuit 22 for further processing, e.g. for supplying the electronic image signal to a hard-copy unit 23 for making a hard-copy of the assembled image.

For performing peripheral angiography, e.g. x-ray imaging of a bloodflow in a patient's leg, a contrast fluid is intra-arterially administered and the progress of the contrast fluid is monitored in that the patient table or the carrier is moved along with the contrast fluid. During the motion of the carrier and/or the patient a plurality of x-ray exposures is performed. The videocamera 13 supplies videosignals for consecutive sub-images corresponding to consecutive x-ray images which are formed on the entrance screen of the image intensifier. The consecutive sub-images are merged into an assembled image by the image processor 20. The blood-flow through the entire leg of the patient can then be examined from one single assembled image that shows a shadow image of the complete leg.

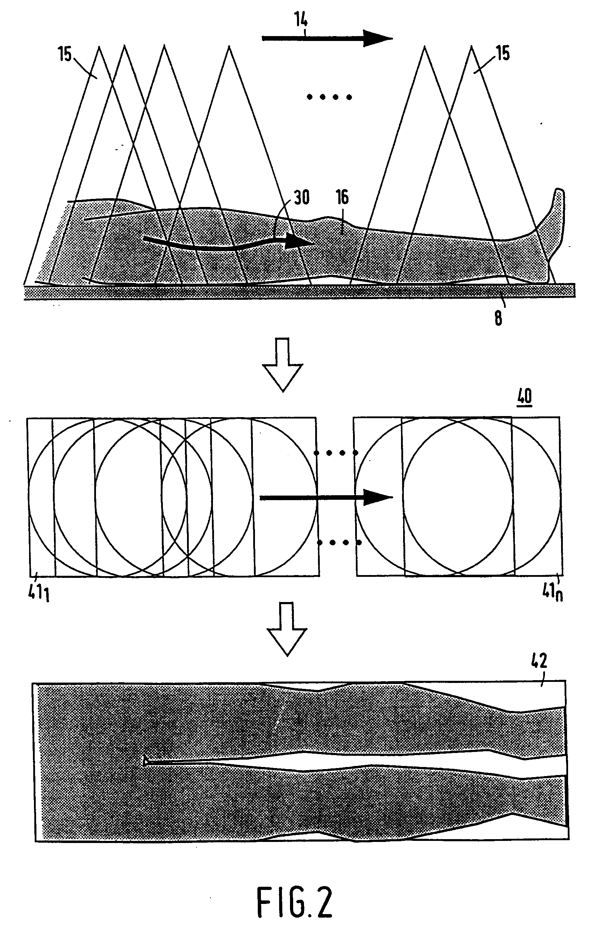

Figure 2 shows a diagrammatic representation of the formation of sub-images and the merging of consecutive sub-images into an assembled image. A patient's leg 16 is shown on the patient table 8. The vertical support 5 is moved along the rails 7, so that the x-ray source is moved in the direction of the arrow 14. As the x-ray source is moved, an x-ray beam 15 is intermittently directed at the patient's leg. The motion of the x-ray source is controlled with respect to the progress of contrast fluid in a blood vessel in the patient's leg as indicated by the arrow 30. Together with the x-ray source the image intensifier is also moved so as to face the x-ray source when the patient is irradiated. This is achieved in that the x-ray source and the image intensifier are mounted to the carrier which is attached to the vertical stand. Whenever the patient's leg is irradiated an x-ray sub-image is formed on the entrance screen of the image intensifier. Thus, a collection 40 is formed of consecutive images $41_1$ to $41_n$ which mutually overlap to various degrees. The overlap between sub-images depends on the displacement between positions of the x-ray source at the irradiation for forming said sub-images. The image processor 20 merges the sub-images of the collection 40 into an assembled image 42 which contains a shadow-image of the entire patient's leg.

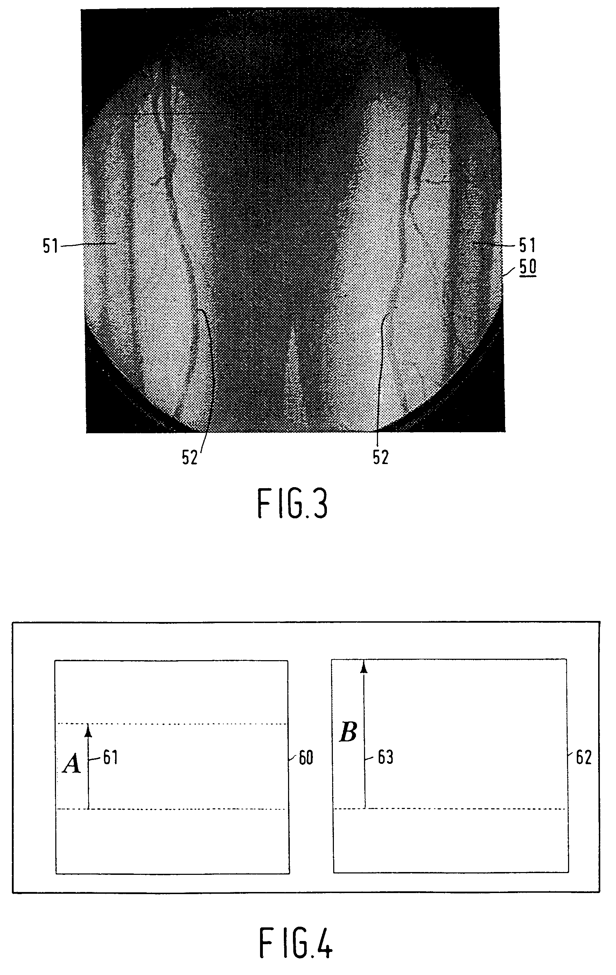

Figure 3 shows an example of a sub-image which is involved in the assembly of an assembled image. A representation 50 of an image registered by the videocamera 13 shows a shadow image of a section at the groin-area of the patient's legs. The image is circular owing to the circular shape of the exit window of the image intensifier. The shadow image features the thigh bones 51 and arteries 52 which are filled with contrast fluid.

Figure 4 illustrates the choice of a gauge vector from a first sub-image and of a template vector from a second sub-image. A patient's limb, e.g. a leg is assumed to be imaged such that the longitudinal axis of the limb is along the column-direction of the pixel-matrices of the consecutive sub-images. A template vector *A* 61 of e.g. 64 pixels in a column of the pixel-matrix 60 is selected in the pixel-matrix 60 of the *N*-th sub-image. In the pixel-matrix 62 of the (*N+1*)-st sub-image a gauge vector *B* of e.g. 192 pixels is selected in a column of the pixel-matrix of the (*N+1*)-st sub-image. The correlation of the template vector with the gauge vector is computed as a function of shift the (*N+1*)-st sub-image with respect to the N-the sub-image. The correlation-value of those two vectors is defined as

$$C_p = |\Sigma_k A_k B_k| / \{[\Sigma_k |A_k|^p]^{1/p} [\Sigma_k |B_k|^{p/(p-1)}]^{(p-1)/p}\}$$

The correlation-value with *p*=2 is found to be a particularly advantageous quantity for determining the mutual shift between successive sub-images. Because of the Cauchy-Schwarz inequality, which is by the way a special case of the Hölder inequality when *p*=2, the correlation-value has the value 1 as its maximum value and attains its maximum value when the template vector perfectly matches with the gauge vector; that is, when the template vector is positioned with respect to the gauge vector so that all pixel-elements of the template vector have the same value as corresponding pixel-elements of the gauge vector. Because of small distortions and noise it may occur that the correlation-value does not reach the value 1. The shift of the (*N+1*)-st sub-image with respect to the *N*-the image is defined as the shift-value for which the correlation-value as a function of the shift-value attains its maximum value. Special portions of the sub-images are seleted for determining the template vector and the gauge vector in order to avoid that the calculation of the shift on the basis of the correlation-value of a template vector and a gauge vector is disrupted because of portions of the image where there is hardly any contrast which would provide the erroneous result that there seems to be no shift at all. Those columns of the pixel-matrices of the sub-images wherein shadow-images of the legs appear are preferably employed for computing mutual shift between consecutive sub-images in the case of peripheral angiography of a patient's legs; in particular with sub-images of 512 columns it is found that virtually any patient's legs are in the columns #40 to #160 and in the columns #352 to #472. These ranges appear adequate for containing shadow images of virtually any adult patient's legs no matter the precise dimensions of the patient who is to be examined. The columns in the ranges of the columns #40 to #160 and the columns in the range #352 to # 472 are selected as the special portions of the sub-images from which the gauge and template vectors are selected. A further refinement is achieved in that the ranges of columns taper towards the patient's feet, so as to select the special portions more in accordance with the patient's anatomy.

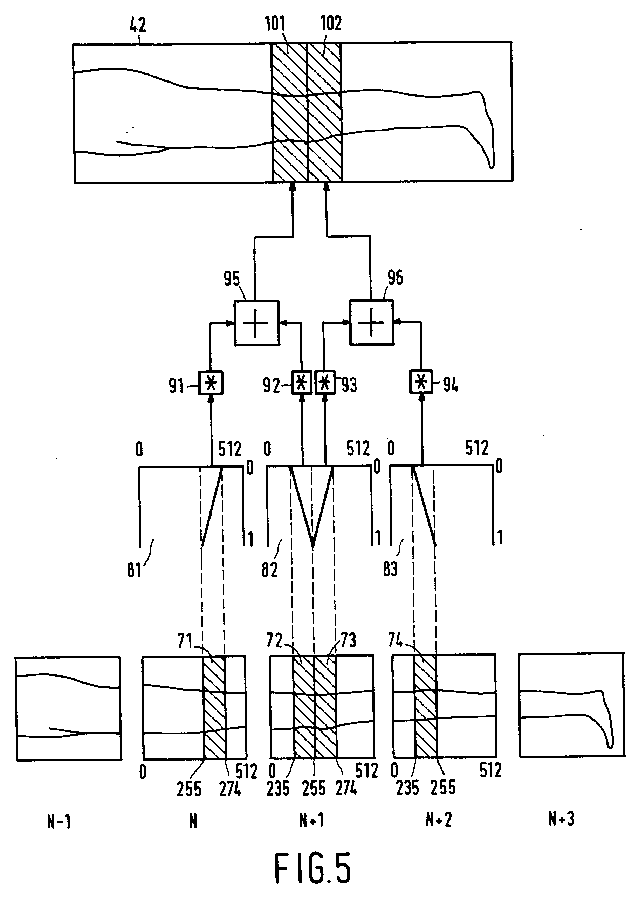

Figure 5 illustrates the merging of sub-images into an assembled image in accordance with the invention. Three consecutive sub-images numbered N, N+1 and N+2 are shown. The scenes in the consecutive images have mutual overlap. The shift between the scenes in one sub-image with respect to the same scene in the next sub-image is computed as discussed hereinbefore in relation to figure 4. The pixel-matrices of consecutive sub-images are arranged such that the shift is perpendicular to the rows of the respective matrices, i.e. the mutual shift is expressed as a number of rows. In each pair of consecutive sub-images, respective sections are selected comprising the same portion of the scene. In each of the sub-images the selected section is located near a central row of that sub-image. Specifically in Figure 5, in the sub-image N the selected section 71 comprises the rows #255 to #274. In the next sub-image, i.e. the sub-image N+1, the rows #235 to #254 are selected as the selected section 72. This example pertains to a pair of consecutive sub-images comprising a mutual shift of 20 rows. Subsequently, the pixels in the selected section 71 in sub-image N are multiplied by a first weight-factor which has a value which decreases from 1 for the first row (#255) of the section 71 to 0 for the final row (#274) of the section 71. The pixels in the selected section 72 in sub-image N+1 are multiplied by a second weight-factor that has a value which increases from 0 for the first row (#255) of the section 71 to 1 for the final row (#274) of the section 71. The first and second weight factors are such that the sum of the first weight factor for a row in section 71 and the second weight factor for a row in section 72 with the same relative position within section 72 as the relative position of said row in section 71 is substantially constant and equals the value 1. Weight factors that vary linearly as a function of the rows in a section are preferred for this. The variation of relevant weight-factors as a function of row-numbers of relevant sections are depicted in the graphs 81,82 and 83. The multiplication of pixel-values by relevant weight-factors is indicated schematically by the multipliers 91,92,93 and 94 and provides weighted pixel-values of rows of sections 71 and 72. Weighted pixel-values of sections 71 are added to weighted pixel-values pertaining to sections 72 such that weighted pixel-values of pixels pertaining to the same position in the scene are added by an addition device 95 each time. The addition of weighted pixels of rows of respective sections produces rows of pixel-values for the assembled image 42. A section 101 of the assembled image is formed from the sections 71 and 72. A section 73 of the sub-image N+1 and a section 74 of the sub-image N+2 are selected in a similar manner, the sub-images 73 and 74 pertaining to the same portion of the scene and said same portion of the scene being slightly shifted with respect to the portion of the scene which is represented in the two sections 71 and 72. Pixel-values of corresponding rows of sections 73 and 74 are multiplied by weight-factors so as to form weighted pixel-values similar to the formation of weighted pixel-values from sections 71 and 72 as discussed hereinbefore. A subsequent section 102 of the assembled image is formed from weighted pixel-values formed from the sections 73 and 74 through addition by an addition device 96 of these weighted pixel-values of the sections 73 and 74 for pixels pertaining to the same position in the scene. The sections 101 and 102 of the assembled image comprise image information on adjacent portions of the scene which is imaged by the camera 13 and which are represented by the sub-images. The sub-images are to be merged into an assembled image representing the entire scene in a single image. Therefore, the section 102 is positioned in the assembled image next to the section 101. To form the entire image, the formation of sections of the assembled image from sections of sub-images which overlap in the scene is re-iterated. Finally, at the extremities of the assembled image, portions of the first and last sub-images of the consecutive series are added. The assembled image then embraces the extremities of the elongate scene, notably the groin-area of the patient and the patient's feet. It is relevant in peripheral angiography of a patient's leg that these extremities are imaged in the assembled image, so that the entire flow of contrast fluid is contained in the assembled image and anatomical orientation in the assembled image is facilitated.

Only image information from sections of sub-images is used in the assembled image, such that in each of the respective sub-images only sections near respective central axes of the relevant sub-images are selected. The rows of the pixel-matrices of the sub-images are arranged perpendicularly to the direction of the mutual shift between the consecutive-sub-images, so that there is almost no parallax error between corresponding rows of consecutive sub-images. Furthermore, the pin-cushion distortion caused by the curvature of the entrance screen of the image intensifier 2 is smaller in portions near the centre of each of the sub-images.

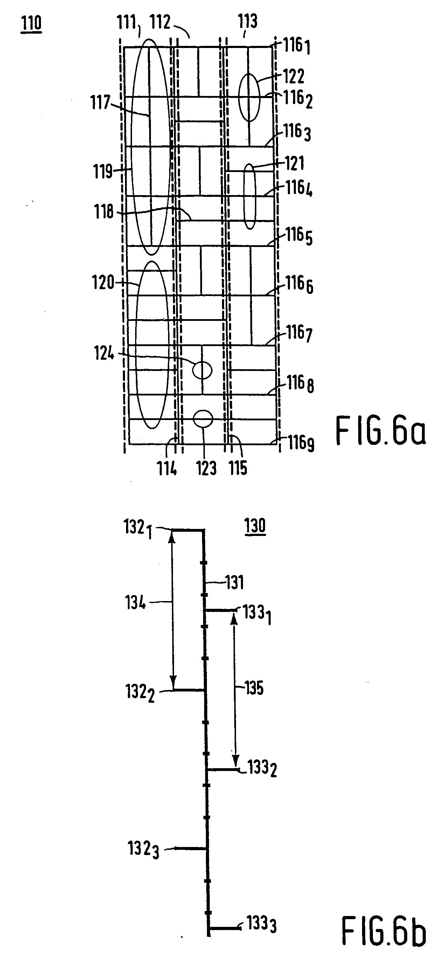

Figures 6a and 6b illustrate respective embodiments of contrasting objects having the shape of a planar ruler with a binary pattern. The ruler of Figure 6a is formed from a steel grid which portionly absorbs x-radiation. The ruler is placed between the patient and the x-ray detector during x-irradiation and consequently a shadow image of the ruler is formed within the shadow image of the patient. The ruler 110 of Figure 6a comprises a pattern of a plurality of columns, three of which 111,112 and 113 are shown. Adjacent columns share a longitudinal bar, explicitly, columns 111 and 112 share the longitudinal bar 114 and the columns 112 and 113 share the longitudinal bar 115. The ruler 110 fur-

ther comprises transverse bars $116_{1-9}$ extending across the width of the ruler. In between adjacent transverse bars there is placed either a bar-section in the longitudinal direction, such as the longitudinal bar-section 117 between the adjacent transverse bars $116_2$ and $116_3$, or a bar-section in the transverse direction, such as the transverse bar-section 118 between the transverse bars $116_4$ and $116_5$, which connects the longitudinal bars 114 and 115. In each of the columns the longitudinal bar sections are arranged in groups which alternate with groups of transverse bar-sections. Column 111 shows one group 119 of longitudinal bar-sections and one group 120 of transverse bar-sections. In column 113 the groups of transverse and longitudinal bar-sections, e.g. 121 and 122 respectively, are alternating with a frequency which is twice the frequency of the alternation in column 111. In column 112 the groups of transverse and longitudinal bar-sections, e.g. 123 and 124, respectively, alternate with a frequency which is twice the frequency of the alternation in column 112. That contrast is thus created in the shadow image with various spatial frequencies so that correlations in consecutive sub-images formed with the use of the ruler 110 are induced at various spatial frequencies. The pattern of the ruler 110 is of binary nature viz. a longitudinal bar-section is interpreted as a 1 and transverse bar-sections is interpreted as a 0, or *vice versa*.

Figure 6b shows another embodiment of a contrasting object in the form of a planar ruler 130. The ruler 130 comprises an elongate central feature, in particular a longitudinal bar 131. From the longitudinal bar 131 first protrusions $132_{1-3}$ extend in a transverse direction. Second protrusions $133_{1-3}$ also extend from the longitudinal bar, in such a way that the longitudinal bar and the first and second protrusions are in the plane of the ruler. In the embodiment depicted here the first and second protrusions extend perpendicularly from the longitudinal bar. However, other embodiments may be constructed where the first and second protrusions enclose oblique angles with the longitudinal bar. The spacing between adjacent first protrusions such as the spacing 134 between the first protrusions $132_1$ and $132_2$ are shifted with respect to adjacent second protrusions such as the spacing 135 between the second protrusions $133_1$ and $133_2$. The binary nature of the ruler 130 is understood by interpreting a first protrusion as a 1 and a second protrusion as a 0, or *vice versa*. Because of the shift between spacings between adjacent protrusions of respective types, the choice of the template vector employed for computing the correlation of adjacent sub-images can be made in such a way that there is always one single protrusion of the same type, i.e. either a single first protrusion and/or a single second protrusion, comprised in the template vector. Consequently, the ruler induces correlations between consecutive sub-images but no spurious correlations pertaining to values of the shift substantially different from the actual shift between consecutive sub-images are induced. When a ruler is employed which has protrusions enclosing oblique angles with the central feature, then spurious correlations are avoided in situations where the series of consecutive sub-image is made while (unintentionally) translating the x-ray detector at an angle with the axis of the elongate scene. A further particular advantage of the ruler 130 is that it adds comparatively small features to the sub-images and the image of the ruler consequently does not substantially deteriorate the diagnostic quality of the assembled image.

The fact that the pattern of both rulers 110 and 130 are predetermined and have a binary nature renders it comparatively simple to delete the image of the ruler from the assembled image by digital electronic post-processing of the assembled image.

Contrasting objects, such as the ruler 110 and the ruler 130 may preferably be incorporated in the patient table.

It is noted that the functions of the image processor may be performed by a suitably programmed computer or by a special purpose processor having circuit means that are arranged to perform said functions in an image processor in accordance with the invention.

**Claims**

1. An image composition method comprising the steps of:

   - making a series of consecutive sub-images ($41_{1-n}$) each representing a portion of an elongate scene, which sub-images overlap along the elongate scene,
   - selecting respective overlapping lengthened portions (71-74) of said sub-images, having respective longitudinal axes substantially transverse to the longitudinal axis of the elongate scene,
   - merging the overlapping lengthened portions into an assembled image (42),
   - interpolating pixel-values of pixels of mutually overlapping lengthened portions of consecutive sub-images which relate to a same position in the elongate scene so as to form pixel-values of the pixels of the assembled image (42) therefrom,
   - determining a correlation between pixel-values of pixels of overlapping portions of the lengthened portions (71-74) as a function of a shift-value between consecutive sub-images,
   - computing the maximum value of said correlation in order to determine a shift with respect to the elongate scene between consecutive sub-images ($41_{1-n}$) through comparison of pixel-values of corresponding pixels of overlapping lengthened portions (71-74) of said consecutive sub-images ($41_{1-n}$), and

- merging the lengthened portions into an assembled image according to said shift.

2. An image composition method as claimed in Claim 1, wherein a contrasting object (110,130) having the form of a ruler comprising a binary pattern is added to the elongate scene, which object intentionally induces correlations in said overlapping portions of said consecutive sub-images.

3. An image composition method as claimed in Claim 2, wherein the ruler (130) has a planar shape and comprises an elongate central feature (131), first protrusions ($132_{1-3}$) extending from the central feature in a first direction and second protrusions ($133_{1-3}$) extending extending from the central feature in a second direction, the pitch (134) between adjacent first protrusions being substantially equal to the pitch (135) between adjacent second protrusions and the spacing between adjacent first protrusions being offset with respect to the spacing between adjacent second protrusions.

4. An x-ray examination apparatus comprising:

- an x-ray source (1) for emitting an x-ray beam (15),
- an x-ray detector (2) facing the x-ray source (1),
- a patient-support (8),
  the x-ray source, the x-ray detector and the patient-support being moveable with respect to one another so as to enable the formation of a series of consecutive sub-images of an elongate portion of the patient by x-irradiation of successive portions of the patient,
- x-ray absorption means which can be arranged between the x-ray source and the x-ray detector in order to attenuate the x-ray beam and to shape the cross-section of the x-ray beam transverse to a central ray of the x-ray beam,
- an image processor (20) for processing the series of consecutive sub-images ($41_{1-n}$) each of them representing a portion of an elongate scene, which sub-images($41_{1-n}$) overlap along the elongate scene, and for merging the sub-images($41_{1-n}$) into an assembled image (42), the image processor comprising :
- an interpolation device (91-96,81-83) for interpolating pixel-values of pixels of overlapping portions of consecutive sub-images so as to form pixel-values of the assembled image therefrom,
- a correlator for determining correlations of pixel-values of pixels of overlapping portions of said consecutive sub-images as a function of a shift-value between consecutive sub-images, and for forming the maximum value of said correlations of pairs of successive sub-images, and
- a field-selector for selecting fields in the form of respective overlapping lengthened portions (71-74) of each of the sub-images having respective longitudinal axes substantially perpendicular to the longitudinal axis of the elongate scene, wherein
  said lengthened portions replace the sub-images ($41_{1-n}$), said merging is performed with the lengthened portions so as to form an assembled image, and the x-ray absorption means are positionable relative to the x-ray beam-path and controlled by the field-selector for selecting the overlapping lengthened portions.

5. An x-ray examination apparatus as claimed in Claim 4, wherein the patient-support (8) incorporates a ruler (110,130) comprising a binary pattern of x-ray absorbing portions and x-ray transmitting portions.

**Patentansprüche**

1. Verfahren zur Bildzusammensetzung, wonach

- eine Anzahl aufeinander folgender Teilbilder ($41_{1-n}$) vorgesehen wird, welche jeweils einen Teil einer verlängerten Ansicht darstellen und sich entlang der verlängerten Ansicht überlappen;
- jeweilige, überlappende, verlängerte Teile (71-74) der Teilbilder ausgewählt werden, welche jeweilige Längsachsen praktisch quer zu der Längsachse der verlängerten Ansicht aufweisen;
- die überlappenden, verlängerten Teile zu einem zusammengesetzten Bild (42) verschmolzen werden;
- Pixelwerte von Pixeln von sich gegenseitig überlappenden, verlängerten Teilen aufeinander folgender Teilbilder, welche die gleiche Stelle in der verlängerten Ansicht betreffen, interpoliert werden, um daraus Pixelwerte der Pixel des zusammengesetzten Bildes (42) vorzusehen;
- eine Korrelation zwischen Pixelwerten von Pixeln überlappender Abschnitte der verlängerten Teile (71-74) als eine Funktion eines Verschiebungswertes zwischen aufeinander folgenden Teilbildern bestimmt wird;
- der Maximalwert der Korrelation berechnet wird, um eine Verschiebung gegenüber der verlängerten Ansicht zwischen aufeinander folgenden Teilbildern ($41_{1-n}$) durch Vergleich von Pixelwerten entsprechender Pixel von überlappenden, verlängerten Teilen (71-74) aufeinander folgender Teilbilder ($41_{1-n}$) zu bestimmen, und

- die verlängerten Teile zu einem zusammengesetzten Bild gemäß der Verschiebung verschmolzen werden.

2. Verfahren zur Bildzusammensetzung nach Anspruch 1, wobei ein kontrastierendes Objekt (110,130), welches die Form eines, ein Binärmuster aufweisenden Lineals besitzt, der verlängerten Ansicht hinzugefügt wird und welches zweckbestimmt Korrelationen in den überlappenden Teilen aufeinander folgender Teilbilder induziert.

3. Verfahren zur Bildzusammensetzung nach Anspruch 2, wobei das Lineal (130) eine planare Form besitzt und ein längliches, mittleres Hauptmerkmal (131), erste Vorsprünge ($132_{1-3}$), welche sich von dem mittleren Hauptmerkmal in eine erste Richtung erstrecken, sowie zweite Vorsprünge ($133_{1-3}$), welche sich von dem mittleren Hauptmerkmal in eine zweite Richtung erstrecken, aufweist, wobei der Zwischenraum (134) zwischen benachbarten, ersten Vorsprüngen dem Zwischenraum (135) zwischen benachbarten, zweiten Vorsprüngen im Wesentlichen gleicht und der Abstand zwischen aneinander grenzenden, ersten Vorsprüngen gegenüber dem Abstand zwischen aneinander grenzenden, zweiten Vorsprüngen gegeneinander versetzt ist.

4. Röntgenuntersuchungsapparant mit:

- einer Röntgenstrahlenquelle (1), um einen Röntgenstrahl (15) zu emittieren,
- einem Röntgendetektor (2), welcher der Röntgenstrahlenquelle (1) gegenüberliegt,
- einer Patientenauflage (8),
  wobei die Röntgenstrahlenquelle, der Röntgendetektor und die Patientenauflage gegeneinander verschiebbar sind, um die Erzeugung einer Anzahl aufeinander folgender Teilbilder einer verlängerten Darstellung des Patienten durch Röntgenbestrahlung sukzessiver Teile des Patienten zu ermöglichen;
- Röntgenabsorptionsmitteln, welche zwischen der Röntgenstrahlenquelle und dem Röntgendetektor angeordnet sein können, um den Röntgenstrahl abzuschwächen und den Wirkungsquerschnitt des Röntgenstrahls quer zu einem Hauptstrahl des Röntgenstrahls zu richten;
- einem Bildprozessor (20), um die Anzahl aufeinander folgender Teilbilder ($41_{1-n}$) zu verarbeiten, welche jeweils einen Teil einer verlängerten Ansicht darstellen und welche sich entlang der verlängerten Ansicht überlappen, und um die Teilbilder ($41_{1-n}$) zu einem zusammengesetzten Bild (42) zu verschmelzen, wobei der Bildprozessor aufweist:
- eine Interpolationsvorrichtung (91-96,81-83), um Pixelwerte von Pixeln von überlappenden Teilen aufeinander folgender Teilbilder zu interpolieren, um daraus Pixelwerte des zusammengesetzten Bildes vorzusehen,
- einen Korrelator, um Korrelationen von Pixelwerten von Pixeln überlappender Teile aufeinander folgender Teilbilder als eine Funktion eines Verschiebungswertes zwischen aufeinander folgenden Teilbildern zu bestimmen und den Maximalwert der Korrelationen von Paaren aufeinander folgender Teilbilder vorzusehen, sowie
- einen Bildfeldselektor, um Bildfelder in Form jeweiliger, überlappender, verlängerter Teile (71-74) jedes der Teilbilder, welche jeweilige Längsachsen praktisch senkrecht zu der Längsachse der verlängerten Ansicht aufweisen, auszuwählen, wobei
  die verlängerten Teile die Teilbilder ($41_{1-n}$) ersetzen, das Verschmelzen der verlängerten Teile durchgeführt wird, um ein zusammengesetztes Bild vorzusehen, und die Röntgenabsorptionsmittel relativ zu dem Röntgenstrahlengang angeordnet sein können und durch den Bildfeldselektor gesteuert werden, um die überlappenden, verlängerten Teile auszuwählen.

5. Röntgenuntersuchungsapparat nach Anspruch 4, wobei in die Patientenauflage (8) ein Lineal (110,130) integriert ist, welches ein Binärmuster aus Röntgenstrahlen absorbierenden Teilen und Röntgenstrahlen durchlässigen Teilen aufweist.

**Revendications**

1. Procédé de composition d'image comprenant les étapes de :

- formation d'une série de sous-images consécutives ($41_{1-n}$) représentant chacune une partie d'une scène allongée, lesquelles sous-images se chevauchent dans le sens de la longueur de la scène allongée;
- sélection de parties (71-74) allongées chevauchantes respectives desdites sous-images ayant des axes longitudinaux respectifs sensiblement transversaux à l'axe longitudinal de la scène allongée;
- fusion des parties allongées chevauchantes en une image assemblée (42);
- interpolation de valeurs-pixels de pixels de parties allongées se chevauchant mutuellement de sous-images consécutives qui se rapportent à une même position dans la scène allongée de manière à former des valeurs-pixels des pixels de l'image assemblée (42) à partir de celles-ci;

- détermination d'une corrélation entre les valeurs-pixels de pixels de parties chevauchantes des parties allongées (71-74) en fonction d'une valeur de décalage entre les sous-images consécutives;
- calcul de la valeur maximale de ladite corrélation afin de déterminer un décalage par rapport à la scène allongée entre des sous-images consécutives ($41_{1-n}$) par comparaison des valeurs-pixels des pixels correspondants des parties allongées chevauchantes (71-74) desdites sous-images consécutives ($41_{1-n}$), et
- fusion des parties allongées en une image assemblée suivant ledit décalage.

2. Procédé de composition d'image suivant la revendication 1, dans lequel un objet de contraste (110, 130) ayant la forme d'une règle comprenant un motif binaire, est ajouté à la scène allongée, lequel objet a pour but d'induire des corrélations dans lesdites parties chevauchantes desdites sous-images consécutives.

3. Procédé de composition d'image suivant la revendication 2, dans lequel la règle (130) a une forme plane et comprend une longue partie centrale (131), des premières saillies ($132_{1-3}$) s'étendant depuis la partie centrale dans une première direction et des deuxièmes saillies ($133_{1-3}$) s'étendant depuis la partie centrale dans une deuxième direction, l'écartement (134) entre les premières saillies voisines étant sensiblement égal à l'écartement (135) entre les deuxièmes saillies voisines, et l'espacement entre les premières saillies voisines étant décalé par rapport à l'espacement entre les deuxièmes saillies voisines.

4. Appareil d'examen à rayons X comprenant :

- une source de rayons X (1) pour émettre un faisceau de rayons X (15);
- un détecteur de rayons X (2) faisant face à la source de rayons X (1);
- un support pour le patient (8),
  la source de rayons X, le détecteur de rayons X et le support pour le patient pouvant être déplacés les uns par rapport aux autres, de manière à permettre la formation d'une série de sous-images consécutives d'une partie allongée du patient par exposition aux rayons X de parties successives du patient;
- des moyens d'absorption de rayons X qui peuvent être disposés entre la source de rayons X et le détecteur de rayons X afin d'atténuer le faisceau de rayons X et de façonner la section du faisceau de rayons X transversalement à un rayon central du faisceau de rayons X;
- une unité de traitement d'image (20) pour traiter la série de sous-images consécutives ($41_{1-n}$), chacune d'elles représentant une partie d'une scène allongée, lesquelles sous-images ($41_{1-n}$) se chevauchent le long de la scène allongée, et pour fusionner les sous-images ($41_{1-n}$) en une image assemblée (42), l'unité de traitement d'image comprenant :

  - un dispositif d'interpolation (91-96, 81-83) pour interpoler les valeurs-pixels de pixels des parties chevauchantes des sous-images consécutives de manière à former les valeurs-pixels de l'image assemblée à partir de celles-ci;
  - un corrélateur pour déterminer les corrélations de valeurs-pixels de pixels de parties chevauchantes desdites sous-images consécutives en fonction d'une valeur de décalage entre des sous-images consécutives, et pour former la valeur maximale desdites corrélations de paires de sous-images successives, et
  - un sélecteur de champ pour sélectionner des champs sous la forme de parties (71-74) allongées chevauchantes respectives de chacune des sous-images ayant des axes longitudinaux respectifs sensiblement perpendiculaires à l'axe longitudinal de la scène allongée,
  dans lequel lesdites parties allongées remplacent les sous-images ($41_{1-n}$), ladite fusion est réalisée avec les parties allongées de manière à former une image assemblée, et les moyens d'absorption de rayons X peuvent être positionnés par rapport au chemin du faisceau de rayons X et contrôlés par le sélecteur de champ pour sélectionner les parties allongées chevauchantes.

5. Appareil d'examen à rayons X suivant la revendication 4, dans lequel le support pour le patient (8) comporte une règle (110, 130) comprenant un motif binaire de parties absorbant les rayons X et de parties transmettant les rayons X.

FIG.1

FIG.2

FIG.3

FIG.4

FIG.5

FIG.6a

FIG.6b